# EUROPEAN PATENT APPLICATION

(11) **EP 2 599 484 A1**
(43) Date of publication of application: **05.06.2013**
(21) Application number: 11803180.6
(22) Date of filing: 04.07.2011
(51) Int. Cl.: A61K 31/195, A61K 9/19

(54) **INJECTABLE PHARMACEUTICAL FORMULATION OF MELPHALAN**

(30) Priority: 05.07.2010 AR P100102398
(71) Applicant: Eriochem, S.A., Parana, Entre Rios (AR)
(72) Inventor: CASTILLO, José, Santa Fe 3000 (AR); ITURRASPE, José Bernardo, Santa Fe - Santafe S3002Foe (AR); NUÑEZ, José Lucio, Paraná - Entre Rios (AR)
(74) Representative: Juncosa Miro, Jaime
(86) International application number: PCT/ES2011/070483
(87) International publication number: WO 2012/004438

(57) **Abstract**

An injectable pharmaceutical formulation of Melphalan comprising a solid composition of melphalan hydrochloride lyophilized with a content of impurities up to 1.3 % (p/p)and a pH buffer solution; a process to prepare said solid composition. Also a reconstituted solution of melphalan comprising a solid composition of melphalan lyophilized reconstituted wherein said solution is aqueous, a perfusion free of organic solvent and a kit.

## Description

### FIELD OF THE INVENTION

The present invention relates to the field of pharmaceutical formulations of sparingly water soluble active principles. In particular, it refers to injectable pharmaceutical formulations of alkylating agents suitable for parenteral infusion processes in oncology chemotherapy.

### STATE OF THE ART

Pharmaceutical formulations of active principles sparingly soluble in aqueous media have been profusely studied over the last decades. Innumerable strategies have been developed in order to be able to inject these active principles into mammals with the aim of improving its pharmacotechnics and diminish its side effects.

Melphalan or p-(bis-2-chloroethyl)-amino-L-phenylalanine or L-sarcolysine, an alkylating agent, cytotoxic, discovered by Bergel et al. in the '50s, and claimed in US patent 3032584, is the active principle of choice in the treatment of several types of cancer.

In the state of the art there have been several attempts to solve the difficulties related to the instability of said drug as well as to achieve directioning aimed at a better effectiveness. Examples that may be mentioned are:

In patent application WO 2008016711 that teaches a composition comprising a salt derivative of pyrimidinecarboxylic acid (Lithium Orotate) and melphalan, the inventors seek to reduce cytotoxic drug levels, such as melphalan in noncancerous tissues by means of derivatizing this drug into a salt of lithium orotate.

US patent 6310039 teaches the formation of a conjugate of transferrin, albumin or PEG, with at least one thiol group (-SH) bound to an anti neoplasic (melphalan), showing a tumor inhibitory effectiveness at least equal or higher than that of the compound alone.

US patent application 20050214310 teaches a method to produce a melphalan prodrug, which may be then activated by an antibody-enzyme conjugate, in which said antibody has specificity for a surface epitope of the tumor cell.

US patent 7135547 teaches a conjugate of glucosaminoglycan bound to melphalan by a linker which presents protease recognition sites.

An example of new formulations is RU patent 2060031, in which a formulation comprising melphalan, polyvinylpyrrolidone, ascorbic acid, glutamic acid, hydrochloric acid and D-mannitol is taught. New injectable formulation tests utilizing cyclodextrin derivatives have been also carried out, as it is described in the work titled "New injectable melphalan formulations utilizing (SBE)7m-β-CD or HP-β-CD" (Int Journal of Pharma Vol 189, Issue 2, 5 Nov 1999, Pages 227-234).

In the state of the art there have been several attempts to develop new formulations of Melphalan proposing combinations of this cytotoxic with other molecule, as taught in patent US 4.738.843, joining Melphalan with immunoglobulin.

Other developments for dosing a great variety of active matter are microparticles which can be obtained from a lyophilization process, such as those described by application US 2005/0238725 which claims a particle smaller than 2000nm and a composition of peptides adsorbed to the surface of the active matter functioning as a tensoactive at the moment of diluting.

Currently, melphalan is commercially available as melphalan tablets containing 2 mg of base melphalan or as lyophilized melphalan hydrochloride together with a reconstitution solution, bearing the trade mark Alkeran®.

The first commercially available formulation of injectable melphalan sterile powder Alkeran® consisted of three components, and it was reconstituted in two stages, just before use. The three components of the system consisted of a vial containing base melphalan, as powder, an ampoule containing acidified alcohol (HCl) and an ampoule containing the solvent-diluent constituted by phosphate buffer diluents and propylene glycol. To reconstitute the injection, melphalan powder was dissolved in the acidified alcohol, stirring until complete dissolution and subsequently melphalan concentration solution was diluted by adding the phosphate buffer and propylene glycol diluent. This three-component system and the two-step reconstitution process were impractical. Moreover, melphalan powder dissolved slowly and in some instances it did not dissolve completely.

The current formulation of lyophilized injectable Alkeran® corresponds to that described in US patent 4997651 by Poole et al. This second injectable formulation of Alkeran® commercially available consists of two components and partially overcomes the disadvantages of the first three-component formulation. The present formulation comprises both lyophilized polyvinylpyrrolidone (PVP) and melphalan hydrochloride, and a solvent diluent comprising a mixture of sodium citrate, water, propylene glycol and ethanol.

Injectable Alkeran® is supplied as a formulation of lyophilized melphalan hydrochloride equivalent to 50 mg of base melphalan and 20 mg of polyvinylpyrrolidone, which is reconstituted in a diluent composed of sodium citrate 0.2 mg, propylene glycol 6.0 ml, ethanol 96% 0.52 mg and water for injection in a quantity sufficient to make 10 ml. In order to be infused, Alkeran® should be diluted up to no more than 0.45 mg/ml in normal saline solution (NSS) 0.9% w/v ClNa and infused for 15 minutes (and 60 minutes before being reconstituted according to its package insert).

Melphalan is the active principle of choice in the treatment of several types of cancer, among which multiple myeloma (MM), amyloidosis (AL), ovarian cancer, malignant melanoma, advanced prostate cancer and testicular cancer can be mentioned.

MM is a neoplasic proliferation of the plasmatic cells characterized by bone lytic lesions, anemia and increase of globulin in serum and urine (3). There are two treatments of MM: group A comprises those who can be subject to an autologous bone marrow transplantation (ATBM), and group B: those who do not have the option to undergo ATBM. Treatment for group A comprises a high dose of melphalan, administered intravenously, followed by ATBM. The standard treatment for group B is melphalan and prednisone (4). The treatment for group B has not changed since it was designed the '60s (5). The treatment for group A started as a study phase I, using a high dose of melphalan (180 mg/m² divided in 3 daily dose of 60 mg/m²) in the mid-80s, and it is becoming one of the most frequently used regimes in combination with ATBM (2). High dose of melphalan followed by ATBM improve response rate and increase survival, and it has become the choice of patients of age 65 or younger, (4) and it is currently the standard treatment for many patients with MM (18). Based on clinical studies, numerous scientific papers show a higher survival rate among patients with MM or light chain amyloidosis (LCA) treated with a high dose of melphalan followed by ATBM (6, 2, 7, 8, 9, 10, 11, 12, 13, 14, 15, 16).

Clinical studies with a high dose of melphalan comprise doses ranging from 100 mg/m² (7, 17, 16, 24) to 200 mg/m² (6, 16, 17, 18, 19, 21, 22, 23, 24). In some cases, the 200 mg/m² dose was administered as the single dose(23, 25, 18).

The LCA is the most common amyloidosis in the developed world. It is a clonal plasma cell disorder in which long chain immunoglobulin synthesized by plasma cells polymerize in fibrillar tissue deposits. These deposits cause dysfunction in the organ. In patients who are not being treated, the illness progresses fast, causing failure and the death of the organ (26). The most frequently affected organs are mainly kidneys, followed by the heart and liver (27). Nowadays, high doses of melphalan with ATBM constitute the "golden standard" for the treatment of chosen patients, since this treatment causes the fastest complete hematological response and organ response (26).

El propylene glycol, CAS 57-55-6, is a clear, viscose and practically odorless liquid. It is very much used in pharmaceutical formulations as solvent, antimicrobial preservative, moisturizer, stabilizing agent and water-miscible cosolvent (28).

Propylene glycol has been identified by the American Academy of Pediatrics as a potentially dangerous additive (29). Even though propylene glycol is considered to be harmless in high concentrations it causes lactic acidosis and hyperosmolarity, hemolysis, renal toxicity including tubular dysfunction and acute tubular necrosis (29, 30).

The World Health Organization (WHO) recommends a total load of 1.875 g of propylene glycol a day for a person who weighs 75 kg (31, 34). Renal damage was determined related to propylene glycol in patients who received 52 g/day, 87.7 g/day and 89.97 g/day of propylene glycol (31).

A 16-year-old child who received daily infusions of phenobarbital and pentobarbital formulations containing propylene glycol for 14 days, being the daily dose of the latter between 10 and 90 g, suffered an acute renal failure caused by propylene glycol. His/The kidney biopsy revealed damage to the proximal tubule cells. It is estimated that propylene glycol, which is lipophilic, penetrates the lipid cell membrane and inside cells it increases osmolality. This causes a flow of liquid towards the interior of cells, causing in turn their swelling and lysis (32).

When propylene glycol is present in high doses in intravenous drugs, it increases the formulation osmolality, and its use, mainly in patients of diminished renal function, should be monitored by determining plasmatic osmolality daily (33).

Formulations containing propylene glycol should not be used in patients with a serious renal dysfunction (35).

Reducing osmolality in parenteral formulations at physiological levels and reevaluating the use of propylene glycol in said formulations has been suggested (36).

The quantity of propylene glycol injected into a patient of MM of a surface of 1.9 m², which corresponds to a person who weighs 75 kg and is 1.75 m tall, with the conventional intravenous melphalan dose stated in Alkeran^{®} package insert, which is 16 mg/m², is 3.648 g, thus being 1.95 times the recommended quantity by the WHO. However, for the treatments of MM and LCA with ATBM, which are not declared in the section of Alkeran^{®} package insert, the dose being imposed as a standard is between 100 - 200 mg/m². In the first case the patient has a surface of 1.9 m² and receives 22.8 g, and in the second one 45.6 g of propylene glycol, which represent 12 and 24 times what is recommended by the WHO. Between 12 - 45 % of propylene glycol is excreted by kidneys, and the rest is metabolized by the liver into lactic acid, piruvic acid and acetone. The half plasma life of propylene glycol in individuals with normal renal function is 5 hours (31).Therefore, most part of propylene glycol administered in high dose regimes will not be metabolized fast, causing hyperosmolality and acidosis, worsening the renal system dysfunction.

The state of the art regarding malphalan injectable formulations and its related problems has been recently described in patent application WO2009150278A1. This document, in its examples number 11 and 12, describes a melphalan injectable formulation at 50 mg/ml of hydrochloric acid 6 N free of propylene glycol, where each ml of it, diluted in 75 ml of normal saline solution is neutralized with 35 ml of a neutralizing aqueous solution of sodium hydroxide, sodium citrate and ethanol.

Alkeran^{®} formulation shows a high tonicity or osmolality of the infusion when administered, which allows -to the utmost- using it in concentrations of 0.45 mg/ml in the infusion, since its osmolality is higher than 1000 mOsmol/kg, which is hyperosmotic. Since plasma osmolality is approximately 290 mOsm/Kg, the infusion solutions of an osmolality between 285 and 310 mOsm/Kg are called isosmotic, and those of an osmolality above 310 are considered to be hyperosmotic. Intravenous administration of hyperosmotic solutions causes shrinkage of blood cells (37). In practice, great ranges of osmolality of intravenous solutions can be tolerated. However, it is also true that achieving an isotonic formulation should constitute an aim for all injectables (38). It is said that the osmolality of parenteral infusions should not exceed 700-800 mOsmol/Kg (39). In practice, the hyperosmolality of the solution of Alkeran^{®} infusion pharmaco-technically does not allow administrations at concentrations above 0.45 mg/ml (1023 mOsmol/kg), even though there are clinical studies using Alkeran^{®} 1 mg/ml, of a really high hyperosmolality of 1971 mOsmol/kg, since this allows using 2.2 times less fluid volume to inject, with the subsequent benefit of a faster administration.

Alkeran® formulation presents a remarkable instability of the 5 mg/ml reconstitution. In the section Preparation for the Administration/Stability of the package insert it is stated that injectable Alkeran® must be reconstituted promptly injecting 10 ml of diluent, stirring vigorously until a clear solution is obtained, to transfer the reconstituted drug in the infusion bag of the normal saline solution and homogenize it by stirring. The reconstituted solution of 5 mg/ml produces a solution with 60% of organic solvents, oversaturated in base melphalan that between 3-10 minutes after preparation produces a precipitate of base melphalane, which cannot be redisolved by stirring and that in such a case must be discarded. The solubility and stability to aqueous hydrolysis of a melphalan aqueous solution depends on its pH and chloride ion concentration. To inject lyophilized melphalan it must be dissolved to a concentration of the order 5 mg/ml, in which an almost completely organic solvent must be used if a pH close to 6, as Alkeran® uses, is desired (EP0317281A2 and US4997651A).

On the other hand, the manufacturing of Alkeran® lyophilisate poses the problem that the acid aqueous solution of the melphalan hydrochlorate is unstable and obligatorily requires manufacturing and dosage at temperatures below dew point in an sterile area, with the resulting pharmaco-technical and regulatory problem of the handling of flasks containing solution, which condensate ambient humidity in their exterior before being filled in the lyophilizer, and they are a potential source of microbiological contamination. The chemical instability of its mother solution to be lyophilized of a generic product of Alkeran®, makes the presence of hydrochloric acid necessary in at least 4 equivalents of hydrochloric acid for each melphalan mol, preferably more than one equivalent and a half of hydrochloric acid for each melphalan mol. The preparation of the mother solution to be lyophilized, of a product equivalent to Alkeran, requires the presence of hydrochloric acid for two reasons in the same sense. Solubility as well as stability increase with the increase of the relative quantity of hydrochloric acid used. Therefore, to dissolve base melphalan in an acceptable time three moles of hydrogen chloride for each melphalan mol are needed, which in turn grant enough stability to perform the production operations of a lot of commercial size without the risk of degradation. Besides, the presence of hydrochloric acid cannot be raised much further than these values due to corrosion problems that an acid solution can cause in a classified environment and fundamentally in the lyophilizer and its vacuum system, since during the lyophilization process hydrogen chloride in excess is released through this evacuation system. In addition, the compromise reached requires working in an acid medium at low temperatures obligatorily, with an acceptable degradation during the lot manufacturing.

Another problem posed by the elaboration of Alkeran® lyophilized formulation is the high viscosity of reconstituted Alkeran®, which, when vigorously stirred to reconstitute the lyophilizate causes a cloud of bubbles that disappears slowly and interferes with the visual inspection of the total dissolution of the lyophilizate.

This present invention solves the problems of the state of the art described so far.

### BRIEF DESCRIPTION OF THE INVENTION

An injectable pharmaceutical of melphalan, object of this present invention, comprises a solid composition of lyophilized melphalan and a pH regulating solution, and in a preferred embodiment it also comprises a reconstitution composition of said melphalan lyophilizate. Being said reconstitution composition a sterile aqueous solution of sodium chloride in a concentration ranging from 0% to 10%. In an alternative of the present invention said reconstitution composition comprises also an organic solvent at a concentration lower than 50 %, preferably lower than 30 %, more preferably lower than 4 %, even more preferably in a quantity of up to 25 times, preferably lower than 10 times the mass of the reconstituted melphalan lyophilizate; being said organic solvent selected from the group comprised by ethanol, propylene glycol, polyethylene glycol 300, polyethylene glycol 400; polyethylene glycol 600, dimethyl sulfoxide, glycofurol, N-methyl-2-pirrolidone, D,L,α,β-isopropylideneglycerol (Solketal®), acetone, dimethylacetamide, glycerol formal, tetrahydrofurfuryl alcohol, and diglyme (dimethyleter of diethylene glycol), or the mixtures thereof.

In another alternative of the present invention, said reconstitution composition is free from organic solvent.

The melphalan injectable pharmaceutical formulation of the present invention comprises said pH regulating solution that is a buffer in aqueous solution, where said buffer is chosen from the group comprised by trisodium citrate dihydrate, sodium citrate, sodium succinate, sodium malate, sodium acetate, sodium tartrate, and the mixtures thereof; also said pH regulating solution comprises said buffer in a mass ranging from 100 to 300 mg for each 50 mg of melphalan of said lyophilized melphalan solid composition.

On the other hand, in the formulation of the invention said lyophilized melphalan as well as said pH regulating solution are diluted in a container of normal saline solution, and said lyophilized malphalan is reconstituted with reconstitution composition of said lyophilized melphalan and injected in an infusion container. Moreover, said pH regulating solution is injected in the same infusion container.

Another object of the present invention is a solid composition which comprises lyophilized malphalan hydrochlorate soluble in water with a content of impurities of up to 1.3 %(P/P), and which may also contain a lyophilization excipient. Said solid composition is obtainable from the lyophilization of a solution comprising acetic acid, preferably glacial; hydrochloric acid in a concentration of up to 0.6 N, preferably up to 4 moles of hydrochloric acid for each of melphalan to be lyophilized, more preferably between 1 and 3 moles; and base melphalan.

Another object of the present invention is a process to manufacture said solid composition comprising the following steps:
a. dissolve base melphalan in acetic acid solution, preferably glacial, and hydrochloric acid, preferably in an aqueous solution at 37 % p/p, preferably in a molar relationship between said hydrochloric acid and said base melphalan (hydrochloric acid moles/base melphalan moles) lower than 4, more preferably between 1 and 3,
b. homogenize,
c. lyophilize, preferably in multiple vials
where said lyophilization of step c. comprises a period shorter than three days and is performed by freezing at a temperature between -30 and -70°C, after freezing for 5 hours, starting vacuum at the same temperature, then bringing the shelf to -20°C, then 0°C and finally drying at 5°C. Also, a lyophilization cake that could be reconstituted in less than 30 seconds is achieved. Also, the time taken by lyophilization step c takes up to two days and the temperature of the solution being homogenyized in step b., until lyophilization, is kept at an ambient temperature higher than 5 °C, preferably 25°C, without degrading.

Another object of the present invention is a reconstituted melphalan solution, preferably of a viscosity below 10 cp, more preferably 5 cp, even more preferably about 1 cp., comprising a reconstituted lyophilized melphalan solid composition in a concentration between 1 and 20 mg/ml, preferably between 3 and 10 mg/ml, more preferably 5 mg/ml, in a reconstitution composition and said solution is aqueous. In addition, this reconstituted solution of the invention comprises a molar relationship between said hydrochloric acid and said base melphalan (hydrochloric acid moles/base melphalan moles) lower or equal than 4, preferably between 1 and 3. Also, said reconstituted solution of the invention comprises a physical stability in the absence of precipitates of at least 3 hours. And said reconstitution composition comprises sterile aqueous solution of sodium chloride in a concentration between 0% and 10%.

In an alternative of the present invention said reconstituted solution said reconstitution composition also comprises an organic solvent in a concentration lower than 50 %, more preferably lower than 30 %, more preferably lower than 4 %, even more preferably in a quantity of up to 10 times the mass of the melphalan lyophilizate being reconstituted; being said organic solvent chosen from the group comprised by ethanol; propylene glycol, polyethylene glycol 300, polyethylene glycol 400; polyethylene glycol 600, Dimethyl sulfoxide, glycofurol, N-methyl-2-pirrolidone, D,L,α,β-isopropylideneglycerol(Solketal®), acetone, dimethylacetamide, glycerol formal, tetrahydrofurfuryl alcohol,and diglyme (dimethyleter of diethylene glycol), or the mixtures thereof.

In a preferred alternative of the present invention said reconstituted solution is free of organic solvent.

Besides, in the reconstituted solution of the invention, said melphalan comprises total impurities lower than 4 % and after 5 minutes of being reconstituted, and said melphalan comprises purity of at least 96 % regarding base melphalan before lyophilization.

Another object of the present invention is a melphalan injectable perfusion comprising melphalan, a pH regulator and a pH between 4 and 6, with an osmolality lower than 320 mOsmol/kg. Where said perfusion, in a preferred embodiment is free of organic solvent. Whereas in an alternative of the present invention said perfusión also comprises organic solvent in a mass relationship regarding said melphalan (organic solvent mass/melphalan mass) lower than 25, preferably lower than 10, preferably lower than or equal to 5.

Also, said perfusion comprises a melphalan concentration between 0.3 and 3 mg/ml, preferably between 0.4 and 2 mg/ml, more preferably around 1 mg/ml.

Another object of the present invention is a kit to elaborate the formulation of claim 1 characterized by comprising a container with lyophilized melphalan solid composition and a container with a regulating pH solution. In an alternative of the present invention said kit to elaborate the formulation of claim 1 comprises a container with the solid composition of lyophilized melphalan, a container with the pH regulating solution and a container with the reconstitution composition of said lyophilized melphalan.

### DETAILED DESCRIPTION OF THE INVENTION

An object of the present invention is a process of non-aqueous lyophilization of base melphalan or its hydrochlorate in acetic acid, preferably glacial, with three moles of concentrated hydrochloric acid or hydrogen chloride for each mol of base malphalan. Where there is a degradation of the API melphalan used, by HPLC (Pharmaeuropa, Vol 19, N° 2, April 2007, pages 318-21), lower than 0.2% in area. As an alternative of the object of the invention, it is hereby presented a lyophilization of melphalan hydrochlorate in glacial acetic acid, with the optional addition of inert excipients such as providone (Kollidon K12).

This same process may be used to obtain melphalan hydrochlorate, which is more convenient than the technique described in Patent US4997651 which uses a concentrated solution of melphalan and hydrogen chloride in ethanol, which is heated for less than five minutes and later cooled to crystallize melphalan hydrochlorate, since the ethanol present in the precipitated melphalan hydrochlorate tends to react and produce melphalan ethylic ester as an impurity in its drying process in an order not below 1%, whereas acetic acid with hydrochloric acid or hydrogen chloride do not pose this problem, and it can be obtained a melphalan hydrochlorare complying with the very strict quality standard established for this API in Pharmaeuropa, Vol 19, N° 2, April 2007, pages 318-21.

To investigate the possibility of elaborating malphalan lyophilizates from pharmaceutical organic solvents, it was determined that base melphalan as well as its hydrochlorate are not soluble in terbutylic alcohol. Instead, even though base malphalan is not soluble in DMSO, its hydrochlorate is soluble up to close to 10 % p/v, prepared by the addition of three moles of hydrochloric acid as a concentrated acid at 37%.

Base melphalan is soluble in glacial acetic acid, preferably free of acetic anhydride, in up to close to 10 % p/v; likewise as melphalan hydrochlorate, mixtures of acetic acid and aqueous solution of hydrochloric acid at 37% p/p to dissolve base melphalan may also be used, or else hydrogen chloride solution in glacial acetic acid.

Base melphalan is stable in a solution of 10 mg/ml of acetic acid. It has been demonstrated by HPLC that it is perfectly stable (see examples), during the hours it must remain in solution while the solution is elaborated and fractionated at ambient temperature, even though after the lyophilization process, preferably if it has been dried at a temperature above 5°C, as for example 25°C, an unacceptable percentage of impurity G is formed. Its results are detailed in the examples.

Melphalan hydrochlorate, which is prepared by the addition of three moles of hydrochloric acid as acid concentrated at 37%, for each mol of malphalan in the solution of 10 mg/ml of melphalan in acetic acid, it has been demonstrated by HPLC (see examples) which is perfectly stable at 25°C, during the hours it must remain in solution while the solution is elaborated and fractionated at ambient temperature, as during and after the lyophilization process, preferably if it has been dried at a temperature not above 5°C. Given that malphalan hydrochlorate, if dried in vacuum of the order of 25 microbars and temperatures higher than 5°C, tends in its lyophilization from acetic acid to lose part of its 2.5 moles of hydrogen chloride per mol of melphalan which composes it, in a greater quantity than its aqueous lyophilization process being able to remain with 1.8 to 1.3 molecules of hydrogen chloride per melphalan molecule, thus originating a melphalan hydrochlorate lyophilize partially soluble in water (pH of an aqueous solution to 5 mg/ml of melphalan hydrochlorate with 2.5 molecules of hydrogen chloride per melphalan mol, which is approximately 1.9). Results are detailed in the examples.

Melphalan hydrochlorate liophilized from DMSO, prepared by the addition of three moles of hydrochloric acid as concentrated acid at 37%, for each mol of melphalan is not very stable since it tends to generate close to 3.6 % increase in total impurities, increasing several of known impurities and adding other unknown ones in its study by HPLC, according to examples.

Melphalan hydrochlorate liophilized from water as shown in Example 6,1,2) requires keeping the aqueous solution of melphalan hydrochlorate at close to 5°C, to avoid hydrolysis problems and its lyophilization is a long and complicated process. Since lyophilizing solutions of hydrochloric acid have a kinetic complication at sublimation of this hydroxy acid from aqueous solution which tends to generate "puffing" problems when the acid is concentrated in the aqueous phase during sublimation, forming an aqueous plastic solid, for it does not sublimate from surface, but boils rendering lyophilization complicated. Whereas lyophilization of melphalan hydrochlorate from glacial acetic acid is a process which that not require keeping its mother solution at low temperatura and its lyophilization and secondary drying can be performed at least in half the time needed for the aqueous process, with a significant decrease in the lyophilization costs. An industrial lot of 5000 units of a generic Alkeran® 50 mg requires 6 days of lyophilizations, whereas from acetic acid it takes two days.

A reconstituted solution of melphalan, object of the present invention, which comprisis melphalan in aqueous solution in an concentration between 1 and 20 mg/ml, preferably between 5 and 10 mg/ml, and more preferably 6.25 mg/ml of lyophilized melphalan, as a hydrochlorate with 1 to 3, preferably 2 molecules of hydrogen chloride for each molecule of melphalan. This new reconstituted solution offers a greater physical and chemical stability than that reconstituted of Alkeran® at 5 mg/ml.

In several alternatives of this invention, the lyophilizate to give the solid composition of lyophilized melphalan hydrochlorate solid composition of the invention, is done optionally from the acetic acid and the hydrochloric acid or their aqueous mixtures, or only water with or without the presence of pharmaceutical organic solvents as excipients.

An injectable pharmaceutical formulation of melphalan, object of the present invention comprises a solid composition of lyophilized melphalan hydrochlorate and a pH regulating solution, which in an alternative also comprises a reconstitution composition of said lyophilized melphalan. Said lyophilized melphalan solid composition is added with normal saline solution or said reconstituent composition; this reconstituted solution is injected in a normal saline solution, then the content of said bag is neutralized with said aqueous pH regulating solution of a stopper agent or buffer, such as sodium citrate, sodium succinate, sodium malate, sodium acetate and sodium tartrate or the mixtures thereof. This pH regulating solution is added to the saline solution before, after or together with said melphalan reconstituted solution to produce a perfusion, also object of the present invention, which is a solution for isotonic infusion of melphalan between 0.3 and 3 mg/ml, preferably between 0.4 and 2 mg/ml and more preferably to 1 mg/ml, with a pH between 4 and 6.

The pH regulating solution which also has the function of a neutralizer is constituted by a pharmaceutically acceptable base or buffer, able to correct the pH of the infusion bag to a value ranging from 4 to 6.

In a preferred embodiment, the solution of the infusion or perfusion is prepared as follows: said lyophilized melphalan solid composition coming from acetic acid, its aqueous mixtures or water, which contains melphalan hydrochlorate and optionally 0.4 mg of povidone K-12 for each mg of melphalan, and upp to 4 molecules of hydrogen chloride for each molecule of melphalan, is reconstituted using water or normal saline solution as a diluent at a concentration of 6.25 mg of melphalan/ml. This reconstituent solution is diluted in normal saline solution to obtain a concentration from 0.45 to 1 mg/ml. This solution is neutralized by the addition of a pH regulating solution of a concentration of 100 mg/ml of trisodium citrate dehydrate in water or in normal saline solution which contains 4 mg of trisodium citrate dehydrate for each mg of melphalan.

The physical stability of said reconstituted solution of melphalan hydrochlorate 6.25 mg/ml of the present invention is of at least of 3 hours, versus the reconstituted solution of the original that starting from some minutes after reconstitution to 5 mg/ml shows suspended particles.

Another relevant aspect of the present invention is the viscosity of said melphalan reconstituted solution. The viscosity determined by method 2.2.9 "Capillary viscometer method" of the European Pharmacopoeia 6.0 the melphalan reconstituted solution of 5 mg/ml of a generic product of Alkeran® was 10.77 cp, whereas the viscosity of the reconstituted solution of 6.25 mg/ml of the present invention is approximately 1 cp.

In the melphalan reconstituted solution no bubbles are observed and the solution is clear immediately after stirring due to its low viscosity.

The osmolality of said perfusion of 0.45 mg/ml of the present invention is more than 3 times lower than that of the original, and if compared with the infusion of 1 mg/ml is almost 6 times lower.

The chemical stability of the infusion of 0.45 and that of 1 mg of melphalan/ml in normal saline solution of the infusion solution of the present invention is similar to that of the original.

The reconstituted melphalan solution of the present invention is obtained, in a preferred embodiment, injecting promptly 8 ml of normal saline solution or water for injectables to the lyophilizate, which contains melphalan hydrochlorate equivalent to 50 mg of melphalan and 20 mg of povidona K-12, followed by vigorous stirring until a clear solution is obtained.

The perfusion of the present invention presents a quantity of organic solvents in an embodiment which does not exceed a mass relationship regarding said melphalan (organic solvent mass/melphalan mass) a value of 10, preferably 5. Therefore, a 50 mg vial of melphalan corresponds to 250 mg of organic solvent,without generating a toxicological problem, which changes this solvent from its category of excipient to the category of a pharmaceutically active product. Thus, when melphalan is infused at 0.45 at 1 mg/ml in normal saline solution, this infusion solution may contain from 2.25 to 5 mg/ml of these solvents useful for pharmaceutical injectables, without generating a toxicological problem in a regime of 200 mg/m².

Said solvent is chosen from a group comprised by ethanol, propylene glycol, polyethylene glycol 300, polyethylene glycol 400; polyethylene glycol 600, Dimethyl sulfoxide, glycofurol, N-methyl-2-pirrolidone, D,L,α,β-isopropylideneglycerol (Solketal®), acetone, dimethylacetamide, glycerol formal, tetrahydrofurfuryl alcohol, and diglyme (dimethyleter of diethylene glycol), or the mixtures thereof.

The desired quantity of organic solvents which may be accepted in a melphalan formulation is related to the high dose regimes of melphalan used in the therapy of bone marrow transplantation, which generally are 200 to 240 mg/m². Thus, when considered 37 Kg/m² for 200 mg/m², it is obtained 5.41 mg melphalan/Kg and due to the fact that the solvents for injectables are in the order of 25 to 50 mg/Kg-day for the WHO, resulting in the fact that a melphalan formulation, within such parameters, may contain up to 5 to 10 parts in weight of a solvent for each part of melphalan.

### EXAMPLES

Purity determinations of melphalan in its different compositions were analyzed by HPLC applying the method Pharmaeuropa, Vol 21, N° 3, July 2009, pages 425-28, for the following examples:

### Example 1

### Lyophilization of melphalan hydrochlorate from acetic acid and its reconstitution and stability assays.

### Example 1.1

428 mg of base melphalan Lot 20-01-2010 was dissolved; purity by HPLC: 96.37%, with impurity B 0% -area-; Imp. C 0.03%; Imp. D 0.12%; Imp. I 0.01%; Imp. J 0.14%; Imp. F 0.02%; Imp. G 2.79%; Imp. H 0.01%; and ethyl ester of melphalan 0.20%; in 43.82 g of a solution of free glacial acetic acid solution of acetic anhydride and 0.415 g of solution at 37 % w/w of aqueous hydrochloric acid and it was homogenized. And 2.0 ml were measured in doses in 23 vials of 5 ml of the glass tube type I y lyophilized in a Virtis Advantage Lyophilizer, freezing at -50°C, after freezing for 5 hours, starting vacuum at the same temperature, then bringing the shelf to -20°C, then to 0°C and finally drying at 5°C obtaining a compact and white cake. Once lyophilization is complete, the vial is stoppered and sealed with an aluminum precinct. Its purity analysis by HPLC was 96.88%, with impurity B 0% -area-; Imp. C 0.05%; Imp. D 0.23%; Imp. I 0.02%; Imp. J 0.14%; Imp. F 0.02%; Imp. G 2.27%; Imp. H 0.01%; and melphalan ethyl ester 0.17%; total weight: 26.3 mg/vial; with 1.3% w/w acetic acid (analyzed by gaseous chromatography) regarding total weight; with a content of 18.7 mg base melphalan per vial, with 15.6% w/w ionizable chloride by USP regarding total weight. This evidences that melphalan hydrochlorate obtained has 1.88 moles of hydrogen chloride per each mol of melphalan. These chromatographic results indicate that this means of lyophilization is appropriate to obtain a lyophylized melphalan that complies with the high purity specifications of Pharmaeuropa, Vol 19, N° 2, April 2007, pages 318-21.

The impurities mentioned in this document are the following:
A: 4-[bis(2-hydroxyethyl)amino]-L-phenylalanine
B: 4-morpholin-4-yl-L-phenylalanine
C: 4-[(2chloroethyl)amino]-L-phenylalanine
D:4-[(2-chloroethyl)(2-hydroxyethyl)amino]-L-phenylalanine
F: 4-[bis(2-chloroethyl)amino]-3-chloro-L-phenylalanine
G: melphalan dimer
J:4-[[2-(2-chloroethoxy)ethyl](2-chloroethyl)amino]-L-phenylalanine

### Example 1.2

To study stability at 25°C of a melphalan solution at 10 mg/ml in glacial acetic acid with 3 moles of hydrochloric acid, from hydrochloric acid at 37% w/we, 940 mg base melphalan Lot 20-01-2010 were used and the results of the following table I were obtained.

| **COMPARATIVE ANALYSIS OF PURITY BY HPLC OF THE STABILITY OF MELPHALAN SOLUTION WITH 3 MOLES OF HCL PER MOL OF MELPHALAN FROM HYDROCHLORIC ACID AT 37% W/W AT 10 mg/ml IN ACETIC ACID AT 25°C VS. ITS API. RESULTS ARE EXPRESSED IN % OF AREA** | | | | | | |
|---|---|---|---|---|---|---|
| SUBSTANCE | API INICIAL | T0 | T1 (1h 15 min) | T2 (2hs 25min) | T4 (5hs) | T5 (17hs 30 min) |
| Melphalan Purity | 97.45 | 97.27 | 97.26 | 97.2 | 97.27 | 97.3 |
| Impurity C | 0.02 | 0.02 | 0.02 | 0.02 | 0.02 | 0.02 |
| Impurity D | 0.09 | 0.08 | 0.08 | 0.08 | 0.07 | 0.04 |
| Impurity I | 0.03 | 0.03 | 0.03 | 0.03 | 0.03 | 0.03 |
| Impurity J | 0.11 | 0.11 | 0.11 | 0.11 | 0.11 | 0.11 |
| Impurity F | 0.05 | 0.05 | 0.05 | 0.05 | 0.05 | 0.05 |
| Impurity G | 2.05 | 2.16 | 2.17 | 2.22 | 2.15 | 2.13 |
| Max. Imp. Disposabl | 0.02 (TRR 0.81) | 0.00 (TRR0.86) | 0.01 (TRR0.86) | 0.01 (TRR0.86) | 0.02 (TRR0.86) | 0.05 (TRR 0.86) |
| Other Imp Disposab. | 0.04 (TRR 1.58) | 0.15 (TRR1.49) | 0.15 (TRR1.49) | 0.15 (TRR1.49) | 0.15 (TRR1.49) | 0.15 (TRR 1.49) |
| Total impurities | 2.55 | 2.73 | 2.74 | 2.80 | 2.73 | 2.70 |

Simultaneously, the melphalan solution was filtered and measured in doses to 10 mg/ml in glacial acetic acid with 3 moles of hydrochloric acid from hydrochloric acid at 37% w/we, which used base melphalan Lot 20-01-2010, in 5 ml vials of the tube flask type, 20 mm mouth, containing 2 ml solution, and it was lyophilized in a Virtis Advantage Lyophilizer, freezing at -50°C for 5 hours, starting vacuum at the same temperature, then bringing the shelf to -20°C, then to 0°C and finally drying at 5°C, with a final vacuum of 25 microbars. A compact and white stopper was obtained. The results of the following table show that lyophilized hydrochlorate melphalan does not show degradation of the initial purity of the API used.

**Table II.**

| **COMPARATIVE ANALYSIS OF PURITY BY HPLC OF THE LYOPHILIZATE FROM MELPHALAN ACETIC ACID WITH 3 MOLES OF HCL PER MOL OF MELPHALAN FROM HYDROCHLORIC ACID 37% W/W (DRIED AT 5°C) VS. ITS API** | | | |
|---|---|---|---|
| Related Substances W/W% | API | Sample 1 | Sample 2 |
| Impurity B | ND | ND | ND |
| Impurity C | < 0.05 | 0.05% | 0.06% |
| Impurity D | 0.05% | < 0.05 | < 0.05 |
| Impurity I | < 0.05 | ND | ND |
| Impurity J | 0.05% | 0.05% | 0.05% |
| Impurity F | ND | ND | ND |
| Impurity G | 1.04% | 1.02% | 1.02% |
| Impurity H | ND | ND | ND |
| Max. Imp. Dispos. | 0.05% | 0.06% | 0.07% |
| Total of Impurities | 1.19% | 1.19% | 1.20% |
| **ND:** Undetectable (i.e., not appearing in chromatograms) | | | |
| **< 0.05:** means that it is in the chromatogram but below quantification limit. Thus it must be reported as <0.05 | | | |

### Example 2

### Reconstitution of the solid composition of lyophilized melphalan in Example 1 to obtain a reconstituted solution of the invention.

A vial containing the solid composition of lyophilized melphalan was reconstituted with 3.2 ml of normal saline solution, producing a melphalan solution at 5.40 mg/ml of pH 1.80. An aliquot of 0.8 ml was taken and added with another of 4.2 ml of normal saline solution, producing 0.86 mg/ml of pH 2.33. It was neutralized with 0.20 ml of a solution of 100 mg/ml of trisodium citrate dihydrate, producing a final pH 5.44.

A vial containing the solid composition of lyophilized melphalan was reconstituted with 0.4 ml absolute ethanol in a melphalan solution at 43 mg/ml.

A vial containing the solid composition of lyophilized melphalan was reconstituted with 3.2 ml of an absolute ethanol solution in normal saline solution at 39 mg/ml, producing a melphalan solution at 5.40 mg/ml, of pH 1.66. An aliquot of 0.8 ml was taken and added with another of 4.2 ml of normal saline solution, producing 0.86 mg/ml of pH 2.28. It was neutralized with 0.20 ml of a solution of 100 mg/ml of trisodium citrate dihydrate, producing a final pH 5.49.

A vial containing the solid composition of lyophilized melphalan was reconstituted with 0.2 ml of DMSO HPLC quality, producing a melphalan solution at 86 mg/ml.

A vial containing the solid composition of lyophilized melphalan was reconstituted with 3.2 ml of DMSO HPLC quality in normal saline solution at 39 mg/ml, producing a melphalan solution at 5.40 mg/ml, of pH 1.66. An aliquot of 0.8 ml was taken and added with another of 4.2 ml of normal saline solution, producing 0.86 mg/ml of pH 2.26. It was neutralized with 0.20 ml of a solution of 100 mg/ml of trisodium citrate dihydrate, producing a final pH 5.46.

A vial containing the solid composition of lyophilized melphalan was reconstituted with 0.2 ml of 1-methyl-2-pyrrolidone producing a melphalan solution of 86 mg/ml.

A vial containing the solid composition of lyophilized melphalan was reconstituted with 3.2 ml of 1-methyl-2-pyrrolidone in normal saline solution at 39 mg/ml, producing a melphalan solution at 5.40 mg/ml of a pH 1.69. An aliquot of 0.8 ml was taken and added with another of 4.2 ml of normal saline solution, producing 0.86 mg/ml of pH 2.30. It was neutralized with 0.20 ml of a solution of 100 mg/ml of trisodium citrate dihydrate, producing a final pH of 5.51.

A vial containing the solid composition of lyophilized melphalan was reconstituted with 0.2 ml of propylene glycol producing a melphalan solution of 86 mg/ml.

A vial containing the solid composition of lyophilized melphalan was reconstituted with 3.2 ml of a propylene glycol solution in normal saline solution at 39 mg/ml, producing a melphalan solution at 5.40 mg/ml, pH 1.65. An aliquot of 0.8 ml was taken and added with another of 4.2 ml of normal saline solution, producing 0.86 mg/ml of pH 2.30. It was neutralized with 0.20 ml of a solution of 100 mg/ml of trisodium citrate dihydrate, producing a final pH of 5.49.

A vial containing the solid composition of lyophilized melphalan was reconstituted with 0.2 ml de polyethylene glycol 400;(Lutrol E 400) producing a melphalan solution at 86 mg/ml.

A vial containing the solid composition of lyophilized melphalan was reconstituted with 3.2 ml de polyethylene glycol 400 (Lutrol E 400) in normal saline solution at 39 mg/ml, producing a melphalan solution at 5.40 mg/ml with a pH of 1.64. An aliquot of 0.8 ml was taken and added with another of 4.2 ml of normal saline solution, producing 0.86 mg/ml of pH 2.28. It was neutralized with 0.20 ml of a solution of 100 mg/ml of trisodium citrate dihydrate, producing a final pH of 5.43.

A vial containing the solid composition of lyophilized melphalan was reconstituted with 0.2 ml of dimethylacetamide producing a solution at 86 mg/ml.

A vial containing the solid composition of lyophilized melphalan was reconstituted with 3.2 ml of dimethylacetamide in a normal saline solution at 39 mg/ml, producing a melphalan solution at 5.40 mg/ml of a pH 1.74. An aliquot of 0.8 ml was taken and added with another of 4.2 ml of normal saline solution, producing 0.86 mg/ml of pH 2.29. It was neutralized with 0.20 ml of a solution of 100 mg/ml of trisodium citrate dihydrate, producing a final pH of 5.50.

### Example 3

### Lyophilization of hydrochlorate of melphalan from DMSO and its reconstitution and stability assays.

479 mg of base melphalan Lot 20-01-2010 was dissolved; purity by HPLC: 96.37%, with impurity B 0% -area-; Imp. C 0.03%; Imp. D 0.12%; Imp. I 0.01%; Imp. J 0,14%; Imp. F 0.02%; Imp. G 2.79%; Imp. H 0.01%; and melphalan ethylic ester 0.20%; in 8.768 g of a solution of dimethyl sulfoxide (DMSO) HPLC quality and 0.445 g of solution at 37 % w/w of aqueous hydrochloric acid, and it was homogeneized. It was measured in doses of 0.40 ml (21.9 mg/vial) in 21 vials - glass tube type I- and it was lyophilized in a Virtis Advantage Lyophilizer, frozen at -50°C, after freezing for 5 hours, starting vacuum at the same temperature, then bringing the shelf to -20°C, then to 17°C and finally drying at 25°C and obtaining a yellowish gummy product. Once lyophilization is complete, the vial is stoppered and sealed with an aluminum precinct? Its purity analysis by HPLC was 92.76 %, with impurity B 0.20% -area-; Imp. C 0.43%; Imp. D 0.52%; Imp. I 0.03%; Imp. J 0.14%; Imp. F 1.23%; Imp. G 2.09%; Imp. H 0.02%; and melphalan ethylic ester 0.14%. These chromatographic results show that this means of lyophilization is not suitable to obtain melphalan complying with the high specifications of purity

### Example 4

### Reconstitution of the lyophilized melphalan solid composition from example 3

A vial containing the solid composition of lyophilized melphalan was reconstituted with 3.2 ml of normal saline solution, producing a melphalan solution at 6.84 mg/ml of pH 2.20. An aliquot of 0.8 ml was taken and added with another of 4.2 ml of normal saline solution, producing 1.09 mg/ml of pH 2.20. It was neutralized with 0.20 ml of a solution of 100 mg/ml of trisodium citrate dihydrate, producing a final pH of 5.59.

A vial containing the solid composition of lyophilized melphalan was reconstituted with 0.2 ml absolute ethanol in a melphalan solution at 110 mg/ml.

A vial containing the solid composition of lyophilized melphalan was reconstituted with 3.2 ml of an absolute ethanol solution in normal saline solution at 39 mg/ml, producing a melphalan solution at 6.84 mg/ml, of pH 1.62. An aliquot of 0.8 ml was taken and added with another of 4.2 ml of normal saline solution, producing 1.09 mg/ml of pH 2.22. It was neutralized with 0.20 ml of a solution of 100 mg/ml of trisodium citrate dihydrate, producing a final pH of 5.64.

A vial containing the solid composition of lyophilized melphalan was reconstituted with 0.2 ml of 1-methyl-2-pyrrolidone producing a melphalan solution of 110 mg/ml.

A vial containing the solid composition of lyophilized melphalan was reconstituted with 3.2 ml of 1-methyl-2-pyrrolidone in normal saline solution at 39 mg/ml, producing a melphalan solution at 6.84 mg/ml of a pH 1.65. An aliquot of 0.8 ml was taken and added with another of 4.2 ml of normal saline solution, producing 1.09 mg/ml of pH 2.24. It was neutralized with 0.20 ml of a solution of 100 mg/ml of trisodium citrate dihydrate, producing a final pH of 5.61.

A vial containing the solid composition of lyophilized melphalan was reconstituted with 0.2 ml of propylene glycol producing a melphalan solution of 110 mg/ml.

A vial containing the solid composition of lyophilized melphalan was reconstituted with 3.2 ml of an propylene glycol solution in normal saline solution at 39 mg/ml, producing a melphalan solution at 6.84 mg/ml, of pH 1.61. An aliquot of 0.8 ml was taken and added with another of 4.2 ml of normal saline solution, producing 1.09 mg/ml of pH 2.22. It was neutralized with 0.20 ml of a solution of 100 mg/ml of trisodium citrate dihydrate, producing a final pH of 5.62.

A vial containing the solid composition of lyophilized melphalan was reconstituted with 0.2 ml de polyethylene glycol 400 ;(Lutrol E 400) producing a melphalan solution at 110 mg/ml.

A vial containing the solid composition of lyophilized melphalan was reconstituted with 3.2 ml de polyethylene glycol 400 (Lutrol E 400) in normal saline solution at 39 mg/ml, producing a melphalan solution at 6.84 mg/ml with a pH of 1.61 An aliquot of 0.8 ml was taken and added with another of 4.2 ml of normal saline solution, producing 1.09 mg/ml of pH 2.21. It was neutralized with 0.20 ml of a solution of 100 mg/ml of trisodium citrate dihydrate, producing a final pH of 5.58.

A vial containing the solid composition of lyophilized melphalan was reconstituted with 0.2 ml of dimethylacetamide producing a solution at 110 mg/ml.

A vial containing the solid composition of lyophilized melphalan was reconstituted with 3.2 ml of dimethylacetamide in normal saline solution at 39 mg/ml, producing a melphalan solution at 6.84 mg/ml of a pH 1.68. An aliquot of 0.8 ml was taken and added with another of 4.2 ml of normal saline solution, producing 1.09 mg/ml of pH 2.24. It was neutralized with 0.20 ml of a solution of 100 mg/ml of trisodium citrate dihydrate, producing a final pH of 5.61.

### Example 5

### Lyophilization of melphalan from aceticacid with and without povidone and its reconstitution and stability assays.

### Example 5.1

374 mg of base melphalan, mixture of the following lots were used: 325 mg of Lot G-MEL-1390-09; purity by HPLC: 98.18%, with impurity B 0.01% -area-; Imp. C 0 %; Imp. D 0.70%; Imp. I 0 %; Imp. J 0.36%; Imp. F 0 %; Imp. G 0.50%; Imp. H 0.01%; and ethylic ester of melphalan 0.1% and 67 mg from Lot G-MEL-134-08. Purity by HPLC: 98.37%, with impurity B 0% -area-; Imp. C 0 %; Imp. D 0.11%; Imp. I 0 %; Imp. J 0,07%; Imp. F 0 %; Imp. G 0.76%; Imp. H 0.02% and ethylic ester of melphalan 0.08.

In a beaker of 50 ml with magnetic stirring, 15 ml of glacial acetic acid are added, 374 mg of base melphalan are added slowly, and they are dissolved easily in acetic acid; the beaker containing base melphalan is washed with 0.49 ml of glacial acetic acid and they are added to the melphalan solution in acetic acid.

To prepare the solution to be lyophilized free from PVP: 7.5 ml of the solution were taken using a micropipette and placed in a 25 ml beaker with magnetic stirring. 3.383 ml of glacial acetic acid were added with a micropipette. This solution is filtered with a nylon 0.45 um filter and has a concentration of 16.67 mg/ml of melphalan in glacial acetic acid.

3.0 ml of this solution are placed in a 15 ml glass tube type I vial and a rubber stopper pharmaceutical quality for lyophilization. The solution is lyophilized in a Virtis lyophilizer Advantage model according to the technique in example 1. Once lyophilization is complete, the vial is stoppered and sealed with an aluminum precinct. A compact and white cake was obtained.

To prepare the solution with PVP: 3.604 ml of glacial acetic acid are added using a micropipette in a 5 ml beaker with magnetic stirring; then 77 mg of PVP Kollidon K-12 are added slowly and dissolved. This solution is transferred to the 50 ml beaker containing the melphalan in glacial acetic acid solution in agitation. This solution is filtered with a nylon 0.45 um filter. 3.0 ml of this solution are placed in a 15 ml -glass tube type I-vial and a butyl rubber stopper for lyophilization. The solution is lyophilized in a Virtis lyophilizer Advantage model. Once lyophilization is complete, the vial is stoppered and sealed with an aluminum precinct. Its purity analysis by HPLC was 90.57 %, with impurity B 0.01% -area-; Imp. C 0.17%; Imp. D 0.55%; Imp. I 0.79%; Imp. J 0.28%; Imp. F 0.03%; Imp. G 7.38%; Imp. H 0.02% and ethylic ester of melphalan 0.34%. These chromatographic results show that this means of lyophilization is not suitable to obtain melphalan complying with the high specifications of purity.

### Example 5.2

To study stability at 25°C of a melphalan solution at 10 mg/ml in glacial acetic acid in the absence of hydrochloric acid, 830 mg base melphalan Lot 20-01-2010 were used and the results of the following table were obtained. This solution is not comprised in the present invention, it is only introduced with the aim of comparing this option without hydrochloric acid presenting higher concentration of impurities than the composition of lyophilized melphalan on the invention.

**Table III**

| **COMPARATIVE ANALYSIS OF PURITY BY HPLC OF THE STABILITY OF MELPHALAN SOLUTION AT 10 mg/ml IN ACETIC ACID AT 25°C VS. ITS API. RESULTS ARE EXPRESSED IN % OF AREA.** | | | | | | |
|---|---|---|---|---|---|---|
| SUBSTANCE | API | T0 | T1 (1h 15 min) | T2 (2hs 25min) | T4 (5hs) | T5 (17hs 30 min) |
| Melphalan Purity | 97.45 | 97.9 | 97.17 | 97.17 | 97.1 | 97.22 |
| Impurity C | 0.02 | 0.02 | 0.02 | 0.02 | 0.03 | 0.04 |
| Impurity D | 0.09 | 0.09 | 0.09 | 0.09 | 0.09 | 0.08 |
| Impurity I | 0.03 | 0.03 | 0.03 | 0.03 | 0.03 | 0.03 |
| Impurity J | 0.11 | 0.11 | 0.11 | 0.11 | 0.11 | 0.1 |
| Impurity F | 0.05 | 0.05 | 0.06 | 0.06 | 0.06 | 0.06 |
| Impurity G | 2.05 | 2.24 | 2.25 | 2.23 | 2.25 | 2.03 |
| Max. Imp. Disposabl | 0.04 (TRR 1.58) | 0.14 (TRR 1.48) | 0.14 (TRR 1.48) | 0.14 (TRR 1.48) | 0.14 (TRR 1.48) | 0.12 (TRR 1.48) |
| Other Imp Dispos. | 0.02 (TRR 0.81) | 0.01 (TRR 0.86) | 0.02 (TRR 0.86) | 0.04 (TRR 0.86) | 0.07 (TRR 0.86) | 0.20 (TRR 0.86) |
| Total of Impurities | 2.55 | 2.81 | 2.83 | 2.83 | 2.90 | 2.78 |

Simultaneously, the melphalan solution was filtered and measured in doses to 10 mg/ml in glacial acetic acid, which used base melphalan Lot 20-01-2010, in 5 ml vials of the tube flask type, 20 mm mouth, containing 2 ml solution, and it was lyophilized in a Virtis Advantage Lyophilizer, freezing at -50°C for 5 hours, starting vacuum at the same temperature, then bringing the shelf to -20°C, then to 0°C and finally drying at 25°C, with a final vacuum of 25 microbars. A compact and white cake was obtained. The results of the following table show that lyophilized melphalan, in the absence of hydrochloric acid, shows degradation of the initial purity of the API used around 1% w/w and a remarkable increase of impurity G.

**Table IV**

| **COMPARATIVE ANALYSIS OF PURITY BY HPLC OF THE MELPHALAN LYOPHILIZATE FROM ACETIC ACID (DRIED AT 25°C) VS. ITS API** | | | |
|---|---|---|---|
| Related substances % W/W | API | Sample 1 | Sample 2 |
| Impurity B | ND | ND | ND |
| Impurity C | ND | 0.08% | 0.10% |
| Impurity D | <0.05 | <0.05 | <0.05 |
| Impurity I | ND | ND | ND |
| Impurity J | <0.05 | 0.06%* | 0.26% |
| Impurity F | <0.05 | 0.05% | 0.05% |
| Impurity G | 0.97% | 1.50% | 1.49% |
| Impurity H | ND | ND | ND |
| Max. Imp. Disp. | 0.07% | 0.07% | 0.09% |
| Total Impurities | 1.04% | 1.77% | 1.99% |
| **ND:** Undetectable (i.e., not appearing in chromatograms) | | | |
| **< 0.05:** means that it is in the chromatogram but below quantification limit. Thus it must be reported as <0.05 | | | |
| *This peak is not clear, thus there is a difference with the duplicate | | | |

### Example 6

### 6,1,1) Lyophilization of melphalan hydrochloride from acetic acid.

In a 1 1 glass flask with magnetic stirring, 315 ml of a solution of glacial acetic acid and 3.88 g of solution at 37 % w/w of aqueous hydrochloric acid, with constant stirring until homogeneization were placed. 4.00 g of melphalan Lot 143-09 were added; purity by HPLC: impurity A not detected; Imp. B no detec.; Imp. C no detec.; Imp. D 0.1%w/w; Imp. J no detec.; Imp. F no detec.; Imp. G 0.9 %w/w; Imp. H no detec.; max. imp. unknown 0.1 %w/w; totals impurities 1.2 %w/w), dispersing with agitation until complete dissolution. The solution was filtered by a 0.22 µm membrane to sterilize and free the solution from the presence of any foreign body. The sterile solution was measured in doses aseptically in 4.0±0.1 ml in 15 ml glass tube type I vials, 20 mm mouth. They were previously treated according to standard and validated procedures in the following way: vials were washed in the automatic washer with water for injectables at 70-75°C, and then depyrogenated and sterilized with dry heat In the oven for 180 minutes at 220°C. Once filled with the solution, vials were stoppered with sterilized bromobutyl rubber stoppers. Then they were placed on a tray for lyophilization. The cycle for freezing the product in the Advantage Virtis lyophilizer was performed by freezing at -50°C, after freezing for 5 hours, starting vacuum at the same temperature, then bringing the shelf to -20°C, then to 0°C and finally drying at 5°C; the vial is stoppered and sealed with an aluminum precinct with plastic cap. The product obtained was analyzed to determine titration, purity, vial staunchness (with methylene blue "European Pharmacopeia 6.0 method 3.2.9, pg. 386-7"), aspect (clarity and color of the solution), sterility and bacterial endotoxins. Vials passed the hermeticity test, and all the other tests complied with the product specifications.

Melphalan lyophilized was obtained this way. Purity by HPLC: impurity A no detec.; Imp. B no detec.; Imp. C <0.05 %; Imp. D 0.21%; Imp. J 0.06%; Imp. F < 0.05 %; Imp. G 0.92%; Imp. H <0.05%; max. imp. unknown <0.05%;total impurities del 1.3 %.

### 6,1,2) Lyophilization of melphalan hydrochloride from an aqueous solution with povidone (generic Alkeran).

In a 1 1 glass flask with magnetic stirring, 500 ml of water for injection at 5°C and 6.0 g of hydrochloric acid concentrated PA at 37 % w/w and 2.00 g of povidone Kollidon K12 were added with constant stirring until homogeneization. 5.00 g of melphalan Lot Mel 143-09 were added. Purity by HPLC: impurity A no detec.; Imp. B no detec.; Imp. C no detec.; Imp. D 0.1% w/w; Imp. J no detec.; Imp. F no detec.; Imp. G 0.9 %w/w; Imp. H no detec.; max. imp. unknown 0.1 %w/w; totals impurities 1.2 %w/w), dispersing with agitation up to total dissolution. The solution was filtered by 0.22 µm membrane for sterilization and free the solution from the presence of any foreign body. The sterile solution was measured in doses at 5°C, aseptically in 5.0±0,1 ml in 15 ml glass tube type I vials, 20 mm mouth/opening. They were previously treated according to standard and validated procedures in the following way: vials were washed in the automatic washer with water for injectables at 70-75°C, and then were depyrogenated and sterilized with dry heat in the oven for 180 minutes at 220°C. Once filled with the solution, vials were stoppered with sterilized bromobutyl rubber stoppers. Then they were placed on a tray for lyophilization. The cycle for freezing the product in the Advantage Virtis lyophilizer was performed for 12 h at -50°C, then vacuum was started and taken to -10, 0, 20; 25 and 30°C during 10 h at each temperature. Vials were stoppered and sealed with an aluminum precinct with plastic cap. The product obtained was analyzed to determine titration, purity, vial staunchness (with methylene blue "European Pharmacopeia 6.0 method 3.2.9, pages 386-7"), aspect (solution clarity and color), sterility and bacterial endotoxins. Vials passed the hermeticity test, and all the other tests complied with the product specifications.

Melphalan lyophilized was obtained this way labeled 6-2-09. Purity by HPLC: impurity A no detec.; Imp. B no detec.; Imp. C <0.05 %; Imp. D 0.61%; Imp. J 0.07%; Imp. F < 0.05 %; Imp. G 0.97%; Imp. H <0.05%; max. imp. unknown <0.05%;total impurities of 1.2 %.

### 6,2)To prepare the dissolvent of generic Alkeran®:

In a stainless steel container, adequate volume with agitation, 0.5 1 of water for injection at 35°C were placed, and 100.9 g of sodium citrate were added with constant stirring. Stirring was maintained until total dissolution during 30 minutes. It was cooled until room temperature. 3000 ml of propylene glycol PA and 250 ml of absolute ethanol PA were added, dispersing with agitation up to total dissolution during 30 minutes. The quantity of injectable quality water needed to complete the final volume of 5.00 1 was added, with agitation for 5 minutes until homogenization. The solution was filtered with 0.22 µm membrane to sterilize and free the solution from the presence of any foreign body. The sterile solution was measured in doses in 10.3±0.1 ml in 15 ml glass tube type I vials, 20 mm mouth. They were previously treated according to the standard procedures and validated as follows: vials were washed in the automatic washer with water for injectables at 70-75°C, and then were depyrogenated and sterilized with dry heat en the oven during 180 minutes at 220°C. Once filled with the solution, vials were stopper with bromobutyl rubber stoppers with Teflon sterilized. Vials were stoppered and sealed with an aluminum precinct with plastic cap. Then they were sterilized in autoclave during 40 minutes at 121°C. The product obtained was analyzed to determine vial staunchness (with methylene blue "European Pharmacopeia 6.0 method 3.2.9, pg. 386-7"), aspect (clarity and color of the solution), sterility and bacterial endotoxins. Vials passed hermeticity test, no turbidity nor opalescence were observed in no vial; pH did not vary regarding the value find in the solution before sterilization in autoclave, and all other tests meet product specifications.

A dissolvent generic Alkeran® was obtained with the following pharmaceutical composition:
Propylene glycol USP 31 6.00 ml
Ethanol USP 31 0.52 ml
Sodium citrate USP 31 200.00 mg
Water for injection q.s. 10 ml

### 6,3,1) Preparation of the reconstituting composition of said lyophilized melphalan of the present invention:

In a stainless steel container, adequate volume with agitation, 3.5 1 of water for injection at 35°C were placed, and 36.0 g of sodium chloride were added with constant stirring. The quantity of injectable quality water need to complete the final volume of 4.00 1 was added, with agitation during 5 minutes until homogenization. The solution was filtered by 0.22 µm membrane for sterilize and free the solution from the presence of any foreign body. The sterile solution was measured in doses in 8.1±0.1 ml in 10 ml glass tube type I vials, 20 mm mouth/opening. They were previously treated according to standard and validated procedures in the following way: vials were washed in the automatic washer with water for injectables at 70-75°C, and then were depyrogenated and sterilized with dry heat in the oven during 180 minutes at 220°C. Once filled with the solution, vials were stopper with bromobutyl rubber stoppers with Teflon sterilized. Vials were stoppered and sealed with an aluminum precinct with plastic cap. Then they were sterilized in an autoclave for 40 minutes at 121°C. The product obtained was analyzed to determine vial staunchness (with methylene blue "European Pharmacopeia 6.0 method 3.2.9, pages 386-7"), aspect (clarity and color of the solution), sterility and bacterial endotoxins. Vials passed hermeticity test, no turbidity nor opalescence were observed in vials; pH did not vary regarding the value found in the solution before sterilization in an autoclave, and all other tests meet product specifications.

A dissolvent of our invention was obtained with the following pharmaceutical composition:
Sodium chloride USP 31 48.0 mg
Water for injection q.s. 8.00 ml

### 6,3,2) Preparation of the pH buffer solution of the present invention:

In a stainless steel container, of a suitable volume supplied with agitation, 1.5 1 of water for injection at 35°C were placed, and 200.5 g of sodium citrate were added with constant stirring. Stirring was maintained until complete dissolution. The quantity of injectable quality water needed to complete the final volume of 2.00 1 was added, with agitation during 5 minutes until homogenization. The solution was filtered by 0.22 µm membrane to sterilize and free the solution from the presence of any foreign body. The sterile solution was measured in doses in 2.1±0.1 ml in 5 ml glass tube type I vials, 20 mm mouth. They were previously treated according to standard and validated procedures as follows: vials were washed in the automatic washer with water for injectables at 70-75°C, and then were depyrogenated and sterilized with dry heat en the oven during 180 minutes at 220°C. Once filled with the solution, vials were stoppered with bromobutyl rubber stoppers with sterilized Teflon. Vials were stoppered and sealed with an aluminum precinct, with a plastic cap. Then they were sterilized in an autoclave for 40 minutes at 121°C. The product obtained was analyzed to determine vial staunchness (with methylene blue "European Pharmacopeia 6.0 method 3.2.9, pages 386-7"), aspect (solution clarity and color), sterility and bacterial endotoxins. Vials passed hermeticity test, no turbidity nor opalescence were observed in vials; pH did not vary regarding the value found in the solution before sterilization in an autoclave, and all other tests meet product specifications.

A dissolvent of our invention was obtained with the following pharmaceutical composition:
Sodium citrate USP 31 200.00 mg
Water for injection q.s. 2.00 ml

### 6,4)Physical stability of the melphalan reconstituted solution of the present invention versus generic Alkeran^{®}

Melphalan lyophilized from example 6,1,1 is reconstituted with 8 ml of the normal saline solution from example 6,3,1, stirring the vial vigorously until obtaining a clear solution. This results in a solution of 6.25 mg/ml of melphalan corresponding to our invention.

Melphalan lyophilized from example 6,1,2 is reconstituted with 8 ml of the normal saline solution from example 6,3,1, stirring the vial vigorously until obtaining a clear solution. This provides a solution of 6.25 mg/ml of melphalan corresponding to our invention.

The melphalan lyophilized from example 6,1,2 is reconstituted with 10 ml of the dissolvent from example 6,2), stirring the vial vigorously until obtaining a clear solution. This provides a solution of 5 mg/ml of melphalan corresponding to generic Alkeran^{®}.

Following the method described in state of the art "2.9.20, particulate contamination: visible particles, from European Pharmacopoeia 6.0", physical stability of the reconstituted solution of Alkeran^{®} equivalent to the original was compared with the one of the present invention.

In the reconstitute corresponding to generic Alkeran® particles in solution could be perceived from the very first minutes.

In the reconstituted solution of melphalan from examples 6,1,1 and 6,1,2, corresponding to the present invention after 3 hours of visual inspection, no precipitate could be perceived.

### 6,5) Compared Osmolality of the perfusion of the present invention versus generic Alkeran®

Determinations of osmolality of the infusion solutions of 0.45 and 1 mg/ml of melphalan in normal saline solution of the generic Alkeran® formulation and of the perfusion of the present invention were performed using a Wescor osmometer, model Vapro 5520. With the aim of comparing the differences between the two formulations and eliminating the contribution of the normal saline solution, the osmolality of the saline solution used to prepare the infusion solutions was also determined.

Preparation of the solutions for infusion of the generic Alkeran® formulation. The melphalan lyophilized from example 5,1,2) is reconstituted with 10 ml of the dissolvent from example 5,2), stirring the vial vigorously until obtaining a clear solution. This provides a solution of 5 mg/ml of melphalan of the generic Alkeran® formulation.

To obtain the perfusion of 0.45 mg/ml, 0.45 ml of the reconstitute are taken and diluted in 4.55 ml of normal saline solution. To obtain the solution of 1 mg/ml, 1 ml of the reconstitute is taken and diluted in 4 ml of normal saline solution.

Preparation of the solutions for infusion of perfusion of formulations of the present invention. Melphalan lyophilizates from example 6,1,1 and 6,1,2 are reconstituted with 8 ml of the normal saline solution from example 6,3,1, stirring the vial vigorously until obtaining a clear solution. This provides two solutions of 6.25 mg/ml of melphalan corresponding to the present invention.

To obtain the solution of 0.45 mg/ml, 3.6 ml of each solution of reconstituted melphalan are taken and diluted in 45.5 ml of saline solution, then 0.9 ml of the pH buffer solution from example 6,3,2 are added to each one. To obtain the solution of 1 mg/ml, 1.6 ml of the reconstitute of each one are taken and diluted in 8 ml of saline solution, then 0.4 ml of the pH buffer solution from example 6,3,2 are added.

Results in Table V determine that the osmolality of the solutions for infusion of generic Alkeran® are from three to six times higher than those of the normal saline solution, while those of the present invention are kept isotonic.

**Table V**

| **Formulation** | **Melphalan concentration (mg/ml)** | **Osmolality (mOsmol/kg)** |
|---|---|---|
| **Generic** Alkeran^{®} | 0-45 | 1023 |
| Present invention from lyophilizate 5,1,1) | 0.45 | 308 |
| Present invention from lyophilizate 5,1,2) | 0.45 | 320 |
| **Generic** Alkeran^{®} | 1 | 1971 |
| Present invention from lyophilizate 5,1,1) | 1 | 330 |
| Present invention from lyophilizate 5,1,2) | 1 | 312 |
| Normal saline solution | na | 289 |

**Table V.** Osmolality of the solutions for infusion of generic Alkeran^{®} versus the ones of the present invention and the normal saline solution.

### 6,6)Chemical stability of the lyophilizate reconstituted solution of our invention versus generic Alkeran®

The melphalan solutions corresponding to the present invention from example 6.4 were analyzed by HPLC (41) to determine purity, titration of 6.25 mg/ml melphalan solutions corresponding to our invention and 5 mg/ml melphalan solution, corresponding to generic Alkeran®.

Stability tests were performed, determining titration and purity of melphalan after 5 minutes of preparing the reconstitute solutions.

On testing, before injecting in the HPLC, the reconstitute is filtered by 0.22 um.

In the same Table 2, impurities of API and the ones of the lyophilizates with each reconstituted solution are compared. The result obtained shows that the reconstituted solutions of the present invention are more stable after 5 minutes of preparation than that of generic Alkeran®, while starting from the same API and purity of the similar lyophilized products, taking into account the analytical variability.

The Alkeran^{®} package insert states that the reconstitute should be diluted immediately after prepared with normal saline solution, because the solution precipitates as established in example 6.4.

**Table VI.** Compared chemical stability of the reconstituted solutions after 5 minutes of generic Alkeran® and the ones of the present invention. Total impurities are expressed as % w/w of melphalan titration in the lyophilizate.

**Table VI**

| **Reconstituted Solution** | **Time (minutes)** | **Total Impurities (Area %)** |
|---|---|---|
| Generic Alkeran® | 5 | 4.04 |
| Present invention from lyophilizate 6,1,1) | 5 | 2.31 |
| Present invention from lyophilizate 6,1,2) | 5 | 2.50 |

### 6.7) Comparative summary of the studies of purity by HPLC of the API used, of the lyophilizate of melphalan hydrochloride from water (generic Alkeran®) and from acetic acid and their respective reconstituted lyophilizates.

The analysis in Table VII allows establishing that the methods of lyophilization of melphalan hydrochloride from water and acetic acid can produce lyophilizates of a similar purity, even if the aqueous one requires mantaining the mother solution at low temperature during is preparation and dispensing to avoid impurity D formation. The reconstitution to 6.25 mg/ml at room temperature of the corresponding lyophilizates of melphalan hydrochloride with normal saline solution (NSS) produces also similar degradations after 5 minutes of reconstituting them, while the reconstitution at 5 mg/ml at room temperature of the aqueous lyophilizate with the generic Alkeran®, after 5 minutes of reconstituting, produces almost double degradation than that obtained when normal saline solution is used as reconstituent.

**Table VII:** Individual and total impurities of the API used, of the lyophilizate from examples 6,1,1 y 6,1,2; and their respective reconstituted solutions to 6,25 and 5 mg/ml after 5 minutes reconstituted.

**Table VII**

| **Impurities** | **Impurities in % w/w in API-Mel 143-09 used in examples 6.1.1) and 6.1.2.)** | **Impurities in % Area of the lyophilizate from acetic acid example 6.1.1) Lot 20-01-10** | **Impurities in % Area of the lyophilizate from water example 6.1.2) Lot 02-06-09** | **Impurities in % Area of the reconstituted in NSS at 6.25 mg/mlof the lyophiliz ate from acetic acid example 6.1.1)** | **Impurities in % Area of the reconstituted in NSS at 6.25 mg/mlof the lyophilizate from water example 6.1.2)** | **Impurities in % Area of the reconstituted in Alkeran® generic dissolvent at 5 mg/ml of the lyophilizate from water of the example 6.1.2)** |
|---|---|---|---|---|---|---|
| Impurity A | ND | ND | ND | ND | ND | ND |
| impurity B | ND | ND | ND | ND | ND | ND |
| Impurity C | ND | < 0.05 | < 0.05 | 0.19 | 0.21 | 0.34 |
| Impurity D | 0.1 | 0.21 | 0.61 | 1.10 | 1.14 | 1.13 |
| Impurity J | ND | 0.06 | 0.07 | 0.06 | 0.07 | 0.14 |
| Impurity F | ND | < 0.05 | < 0.05 | < 0.05 | < 0.05 | < 0.05 |
| Impurity G | 0.9 | 0.92 | 0.97 | 0.94 | 0.95 | 1.91 |
| Impurity H | ND | < 0.05 | < 0.05 | < 0.05 | < 0.05 | < 0.05 |
| **Max. Imp. Desc.** | 0.1 | < 0.05 | < 0.05 | 0.11 | 0.13 | 0.14 |
| **Total Impurities** | 1.2 | 1.3 | 1.2 | 2.31 | 2.50 | 4.04 |

It's worth highlighting that due to the deviation from the Beer Law, at low concentrations of impurities in regards to melphalan, the percentages weight in weight %(w/w)are equivalent to the percentages in area % area multiplied by a 0.6 factor.

### 6.8. Presentation of the injectable pharmaceutical invention.

The presentation of the formulation of the present invention as an injectable finished product is a kit of two or three vials, comprising the lyophilizate vial and the neutralizing solution vial. Besides, it can contain the vial of the reconstitution composition of said lyophilized melphalan. In an alternative of the present invention, the lyophilizate is reconstituted with a normal saline solution, thus incorporating it to the kit is not necessary.

The blister of 2 or 3 beds containing the vials is found together with the package insert inside a cardboard package with the corresponding obligatory information.

In an alternative of the present invention said kit comprises a pre-filled syringe.

### REFERENCIAS

1) Prospecto de Alkeran inyectable.
2) Hillar M. Lazarus et al.: High-Dose Melphalan and the Development of Hematopoietic Stem-Cell Transplantation: 25 Years Later. Journal of Clinical Oncology 26, 2240:2243, 2010.
3) Vassallo, J.A.; Barrios, E..: Actualización Ponderada de los Factores de Riesgo del Cáncer. Montevideo: Comisión Honoraria de Lucha contra el Cáncer, 2003.
4) Eduardo Flores Sañudo: Estado Actual del Tratamiento del Mieloma Múltiple. IX Congreso Nacional de la Sociedad Española de Oncologia Médica, Tenerife, 2003.
5) J. Parker, P. Parker: Multiple Myeloma. ICON Health Publications, 2004
6) R. L. Comenzo, E. Vosburgh et al.: Dose-Intensive Melphalan With Blood Stem-Cell Support for the Treatment of AL (Amyloid Light-Chain) Amyloidosis: Survival and Responses in 25 Patients. Blood 91, 3662-3670, 1998.
7) S. Girnius,1 D. C. Seldin: Hepatic response after high-dose melphalan and stem cell transplantation in patients with AL amyloidosis associated liver disease. Haematologica 94, 7, 1029-1032, 2009.
8) H. Goldschmidt, H. Lannert, et al.: Multiple Myeloma and renal failure. Nephrol Dial Transplant. 15, 301-304, 2000.
9) V. Sanchorawala, M. Skinner et al.: Long-term outcome of patients with AL amyloidosis treated with high-dose melphalan and stem-cell transplantation. Blood 110, 10, 3561-3569, 2007.
10) P. Zhou, J. Teruya-Feldstein et al.: Calreticulin expression in the clonal plasma cells of patients with systemic light-chain (AL-) amyloidosis is associated with response to high-dose melphalan. Blood 111, 2, 549-557, 2008.
11) D. Seldin, J. Anderson et al.: Improvement in quality of life of patients with AL amyloidosis treated with high-dose melphalan and autologous stem cell transplantation. Blood 104, 6, 1888-1893, 2004.
12) B. Björkstrand, T. Klausen et al.: Double versus single high-dose melphalan 200 mg/m2 and autologous stem cell transplantation for multiple myeloma: a region-based study in 484 patients from the Nordic area. Hematology Reviews, 1, 1, 2009
13) P. Moreau, T. Facon et al.: Comparison of 200 mg/m2 melphalan and 8 Gy total body irradiation plus 140 mg/m2 melphalan as conditioning regimens for peripheral blood stem cell transplantation in patients with newly diagnosed multiple myeloma: final analysis of the Intergroupe Francophone du Mye'lome 9502 randomized trial. Blood 99, 3, 731-735, 2002.
14) D. Seldin, J. Anderson et al.: Successful treatment of AL amyloidosis with high-dose melphalan and autologous stem cell transplantation in patients over age 65. Blood 108, 12, 3945-3947, 2006.
15) V. SANCHORAWALA, D. SELDIN: An overview of high-dose melphalan and stem cell transplantation in the treatment of AL amyloidosis. Amyloid, 14, 4, 261-269, 2007
16) M. ATTAL, J. HAROUSSEAU et al.: A PROSPECTIVE, RANDOMIZED TRIAL OF AUTOLOGOUS BONE MARROW TRANSPLANTATION AND CHEMOTHERAPY IN MULTIPLE MYELOMA. The New England Journal of Medicine, 335, 2, 91-97, 1996
16) M. Skinner, V. Sanchorawala et al.: High-Dose Melphalan and Autologous Stem-Cell Transplantation in Patients with AL Amyloidosis: An 8-Year Study. Ann Intern Med. 140, 85-93. 2004
17) J. Perz, S. Schonland: High-dose melphalan with autologous stem cell transplantation after VAD induction chemotherapy for treatment of amyloid light chain amyloidosis: a single centre prospective phase II study. British Journal of Haematology, 127, 543-551, 2004.
18) M. Dimopoulos, V. Souliotis et al.: Extent of Damage and Repair in the p53 Tumor- Suppressor Gene After Treatment of Myeloma Patients With High-Dose Melphalan and Autologous Blood Stem-Cell Transplantation Is Individualized and May Predict Clinical Outcome. J Clin Oncol, 234, 381-4389, 2005
19) D. Vesole, J. Crowley et al.: High-Dose Melphalan With Autotransplantation for Refractory Multiple Myeloma: Results of a Southwest Oncology Group Phase II Trial. J Clin Oncol, 17, :2173-2179, 1999
20) G. Sarosy, B. Leyland-Jones et al.: The Systemic Administration of Intravenous Melphalan. Journal of Clinical Oncology, 6, 11, 1768-1782, 1988.
21) N. Blijlevens, M. Schwenkglenks et al: Prospective Oral Mucositis Audit: Oral Mucositis in Patients Receiving High-Dose Melphalan or BEAM Conditioning Chemotherapy-European Blood and Marrow Transplantation Mucositis Advisory Group. J Clin Oncol, 26, 1519-1525. 2008
22) R. Comenzo, E. Vosburgh et al.: Dose-Intensive Melphalan With Blood Stem Cell Support for the Treatment of AL Amyloidosis: One-Year Follow-up in Five Patients. Blood, 88, 7, 2801-2806, 1996
23) S. Lenhoff, M. Hjorth et al.: Impact on survival of high-dose therapy with autologous stem cell support in patients younger than 60 years with newly diagnosed multiple myeloma: a population-based study. BLOOD, 95, 1, 7-11, 2000
24) D. Vesole, B. Barlogie et al.: High-Dose Therapy for Refractory Multiple Myeloma: Improved Prognosis With Better Supportive Care and Double Transplants. Blood, 84, 3, 950-956, 1994
25) J. Child, M. Gareth et al.: High-Dose Chemotherapy with Hematopoietic Stem-Cell Rescue for Multiple Myeloma. N Engl J Med, 348, 1875-83, 2003.
26) D.Seldin: Can AL amyloidosis be cured. XIth International Symposium on Amyloidosis. CRC Press, 2008
27) L. A. Sikkink, E. M. Baden et al.: AGGRESSIVE LIGHT CHAIN AMYLOIDOSIS CASES HAVE UNSTABLE IMMUNOGLOBULIN LIGHT CHAINS. Amyloid and Amyloidosis. CRC Press, 2005.
28) Handbook of Pharmaceutical Excipients, Sixth edition. Pharmaceutical Press, 2009.
29) M. Chicella, P. Cansen et al.: Propylene glycol accumulation associated with continuous infusion of lorazepam in pediatric intensive care patients. Crit Care Med, 30, 12, 2752-2756, 2002
30) Mark G. Parker, Gilles L. Fraser et al.: Removal of propylene glycol and correction of increased osmolar gap by hemodialysis in a patient on high dose lorazepam infusion therapy. Intensive Care Med, 28, :81-84, 2002
31) M. Levy, M. Aranda et al.: Propylene Glycol Toxicity Following Continuous Etomidate Infusion For The Control Of Refractory Cerebral Edema. Neurosurgery. 37, 2, 363-371, 1995
32) P. Yorgin, A. Theodorou et al.: Propylene Glycol - Induced Proximal Renal Tubular Cell Injury. American Journal of Kidney Diseases, 30, 1, pp 134-1 39, 1997
33) H. Demey, R. Daelemans et al.: Propylene glycol-induced side effects during intravenous nitroglycerin therapy. Intensive Care Med, 14, 221-226, 1988
34) M. Khandoker, J. Sushil et al.: Propylene Glycol-Mediated Cell Injury in a Primary Culture of Human Proximal Tubule Cells. TOXICOLOGICAL SCIENCES 46, 410-417, 1998 (1998)
35) M. Cawley. Short-Term Lorazepam Infusion and Concern for Propylene Glycol Toxicity: Case Report and Review. Pharmacotherapy 21, 9, 1140-1144, 2001
36) A. Doenicke, A. Nebauer, et al.: Osmolalities of Propylene Glycol-Containing Drug Formulations for Parenteral Use. Should Propylene Glycol Be Used as a Solvent? Anesth Analg, 75:431-435, 1992
37) M. Stranz, E. Kastango: A REVIEW of pH AND OSMOLARITY. International Journal of Pharmaceutical Compounding, 6, 3, 2002
38) Encyclopedia of PHARMACEUTICAL TECHNOLOGY, Third Edition. Informa Healthcare USA, 2007
39) Remington, 19a edición. Editorial Médica Panamericana, 1998.
40) Masako Ohnishi and Hiromichi Sagitani: The Effect of Nonionic Surfactant Structure on Hemolysis. JAOCS, 70, 7, 679-684, 1993
41) Pharmeuropa, 21, 3, 425-426, 2009.

## Claims

1. An injectable pharmaceutical formulation of Melphalan comprising a solid composition of melphalan hydrochloride lyophilized and a pH buffer solution.

2. An injectable pharmaceutical formulation of Melphalan, according to claim 1, wherein also comprising a reconstitution composition of said melphalan lyophilized.

3. An injectable pharmaceutical formulation of Melphalan, according to claim 2, wherein said reconstitution composition comprises a sterile aqueous solution of sodium chloride at a concentration of between 0% and 10%.

4. An injectable pharmaceutical formulation of Melphalan, according to claim 3, wherein said reconstitution composition also comprises an organic solvent.

5. An injectable pharmaceutical formulation of Melphalan, according to claim 4, wherein said reconstitution composition comprises said organic solvent at a concentration lower than 50 %.

6. An injectable pharmaceutical formulation of Melphalan, according to claim 4, wherein said reconstitution composition comprises said organic solvent at a concentration lower than 30 %.

7. An injectable pharmaceutical formulation of Melphalan, according to claim 4, wherein said reconstitution composition comprises said organic solvent at a concentration lower than 4 %.

8. An injectable pharmaceutical formulation of Melphalan, according to claim 4, wherein said reconstitution composition comprises said organic solvent by an amount until 10 times Melphalan lyophilized mass to be reconstituted.

9. An injectable pharmaceutical formulation of Melphalan, according to claim 4, wherein said organic solvent is chosen from the group comprised by ethanol, propylene glycol, polyethylene glycol 300, polyethylene glycol 400; polyethylene glycol 600, dimethyl sulfoxide, glycofurol, N-Methyl-2-pyrrolidone; D,L,α,β-isopropylidene glycerol (Solketal®), tetrahydrofurfuryl alcohol, and diglyme (diglycol methyl ether), or the mixtures thereof.

10. An injectable pharmaceutical formulation of Melphalan, according to claim 3, wherein said reconstitution composition is free of organic solvent.

11. An injectable pharmaceutical formulation of Melphalan, according to claim 1, wherein said pH buffer solution comprises a buffer in aqueous solution.

12. An injectable pharmaceutical formulation of Melphalan, according to claim 11, wherein said buffer is chosen from the group comprised by trisodium citrate dihydrate, sodium citrate, sodium succinate, sodium acetate, sodium tartrate, and the mixtures thereof.

13. An injectable pharmaceutical formulation of Melphalan, according to claim 11, wherein said buffer comprises a mass of between 100 and 300 mg per each 50 mg of melphalan of said solid composition of lyophilized melphalan.

14. An injectable pharmaceutical formulation of Melphalan, according to claim 1, wherein both said melphalan lyophilized and said pH buffer solution are diluted in normal saline solution.

15. An injectable pharmaceutical formulation of Melphalan, according to claim 2, wherein said melphalan lyophilized is reconstituted with said reconstitution composition of Melphalan lyophilized in the infusion container.

16. An injectable pharmaceutical formulation of Melphalan, according to claim 1, wherein said pH buffer solution is injected in infusion container.

17. A solid composition wherein it comprises Melphalan lyophilized with a content of impurities up to 1.3 % (p/p).

18. A solid composition, according to claim 17, wherein it comprises a water-soluble lyophilizate.

19. A solid composition, according to claim 17, wherein it also comprises an excipient for lyophilization.

20. A solid composition, according to claim 17, wherein it is obtainable from lyophilizing a solution comprising acetic acid, hydrochloric acid and base melphalan.

21. A solid composition, according to claim 20, wherein said acetic acid is glacial and said hydrochloric acid comprises a concentration up to 0.6 N

22. A solid composition, according to claim 20, wherein said hydrochloric acid comprises a concentration up to 4 moles of hydrochloric acid per each mol of Melphalan to be lyophilized.

23. A solid composition, according to claim 20, wherein said hydrochloric acid comprises a concentration of between 1 and 3 moles of hydrochloric acid per each mol of Melphalan to be lyophilized.

24. A process to prepare the solid composition according to claim 17 wherein it comprises the following steps:
a. dissolve base Melphalan in an acetic acid and hydrochloric acid solution,
b. homogenize,
c. lyophilize

25. A process, according to claim 24, wherein the acetic acid in step a is glacial.

26. A process, according to claim 24, wherein said hydrochloric acid added as an aqueous solution at 37 % p/p.

27. A process, according to claim 24, wherein in step a, the molar rate between said hydrochloric acid and said base Melphalan (hydrochloric acid moles/base Melphalan moles) is less than 4.

28. A process, according to claim 24, wherein in step a, the molar rate between said hydrochloric acid and said base Melphalan (hydrochloric acid moles/base Melphalan moles) is between 1 and 3.

29. A process, according to claim 24, wherein said lyophilization is carried out after dosing the dissolution obtained in step b in multiple vials.

30. A process, according to claim 24, wherein said lyophilization in step c achieves a compact cake that can be reconstituted in less than 30 seconds.

31. A process, according to claim 24, wherein the time lasting step c is up to two days.

32. A process, according to claim 24, wherein the temperature of the solution homogenizing in step b, up to lyophilizing is kept at a temperature higher than 5 °C without degradation.

33. A reconstituted solution of melphalan comprising a solid composition of melphalan lyophilized reconstituted wherein said solution is aqueous.

34. A reconstituted solution of melphalan, according to claim 33, wherein it comprises a melphalan concentration of between 1 and 20 mg/ml.

35. A reconstituted solution of Melphalan, according to claim 33, wherein it comprises a melphalan concentration of between 3 and 10 mg/ml.

36. A reconstituted solution of Melphalan, according to claim 33, wherein it comprises a melphalan concentration of about 6.25 mg/ml.

37. A reconstituted solution of Melphalan, according to claim 33, wherein it comprises a molar rate between said hydrochloric acid and said base Melphalan (hydrochloric acid moles/base Melphalan moles) lower than or equal to 4.

38. A reconstituted solution of Melphalan, according to claim 33, wherein it comprises a molar rate between said hydrochloric acid and said base Melphalan (hydrochloric acid moles/base Melphalan moles) of bwtween 1 and 3.

39. A reconstituted solution of Melphalan, according to claim 33, wherein it comprises a viscosity lower than 10 cp.

40. A reconstituted solution of Melphalan, according to claim 33, wherein it comprises a viscosity lower than 5 cp.

41. A reconstituted solution of Melphalan, according to claim 33, wherein it comprises a viscosity of about 1 cp.

42. A reconstituted solution of Melphalan, according to claim 33, wherein it comprises a physical stability in absence of precipitate of at least 3 ours.

43. A reconstituted solution of Melphalan, according to claim 33, wherein said reconstitution composition comprises a sterile aqueous solution of sodium chloride at a concentration of between 0% and 10%.

44. A reconstituted solution of Melphalan, according to claim 43, wherein said reconstitution composition also comprises an organic solvent.

45. A reconstituted solution of Melphalan, according to claim 44, wherein said reconstitution composition comprises said organic solvent at a concentration lower than 50 %.

46. A reconstituted solution of Melphalan, according to claim 44, wherein said reconstitution composition comprises said organic solvent at a concentration lower than 30 %.

47. A reconstituted solution of Melphalan, according to claim 44, wherein said reconstitution composition comprises said organic solvent at a concentration lower than 4 %.

48. A reconstituted solution of Melphalan, according to claim 44, wherein it comprises said organic solvent by an amount until 10 times Melphalan lyophilized mass to be reconstituted.

49. A reconstituted solution of Melphalan, according to claim 44, wherein said organic solvent is chosen from the group comprised by ethanol, propylene glycol, polyethylene glycol 300, polyethylene glycol 400; polyethylene glycol 600, dimethyl sulfoxide, glycofurol, N-Methyl-2-pyrrolidone; D,L,α,β-isopropylidene glycerol (Solketal®), tetrahydrofurfuryl alcohol, y diglyme (diglycol methyl ether), or the mixtures thereof.

50. A reconstituted solution of Melphalan, according to claim 43, wherein it is free from organic solvent.

51. A reconstituted solution of Melphalan, according to claim 43, wherein said Melphalan comprises total impurities lower than 4 % after 5 minutes reconstitution.

52. A reconstituted solution of Melphalan, according to claim 43, wherein said Melphalan comprises a purity of at least 96 % regarding base melphalan before lyophilization.

53. An injectable perfusion of Melphalan wherein it comprises melphalan, a pH buffer and a pH between 4 and 6.

54. A perfusion, according to claim 53, wherein it comprises an osmolality lower than 320 mOsmol/kg.

55. A perfusion, according to claim 53, wherein it also comprises organic solvent in mass rate to said Melphalan (organic solvent mass/ melphalan mass) lower than 25.

56. A perfusion, according to claim 55, wherein it comprises said organic solvent in mass rate to said Melphalan (organic solvent mass/ melphalan mass) lower than or equal to 10.

57. A perfusion, according to claim 53, wherein it comprises a Melphalan concentration between 0.3 and 3 mg/ml.

58. A perfusion, according to claim 53, wherein it comprises a Melphalan concentration between 0.4 and 2 mg/ml.

59. A perfusion, according to claim 53, wherein it comprises a Melphalan concentration of about 1 mg/ml.

60. A perfusion, according to claim 53, wherein it is free from organic solvent.

61. A kit containing the formulation according to claim 1 wherein it comprises a container with the solid composition of lyophilized Melphalan and a container with the pH buffer solution.

62. A kit containing the formulation according to claim 1 wherein it comprises a container with the solid composition of lyophilized Melphalan, a container with the pH buffer solution and a container with the reconstitution composition of said lyophilized Melphalan.
